# FASCICULE DE BREVET EUROPEEN

(11) **EP 2 885 265 B1**
(45) Date de publication et mention de la délivrance du brevet: **11.01.2017**
(21) Numéro de dépôt: 13758951.1
(22) Date de dépôt: 07.08.2013
(51) Int. Cl.: C07C 29/78, C07C 31/26

(54) **PROCEDE DE FABRICATION DE COMPOSITIONS RICHES EN CRISTAUX DE MANNITOL SOUS FORME DELTA**
VERFAHREN ZUR HERSTELLUNG VON DELTA-FORM-MANNITOLKRISTALLENREICHEN ZUSAMMENSETZUNGEN
PROCESS FOR PRODUCING COMPOSITIONS RICH IN CRYSTALS OF MANNITOL IN DELTA FORM

(30) Priorité: 17.08.2012 FR 1257859
(43) Date de publication de la demande: 24.06.2015
(60) Demande divisionnaire: 16200314.9
(73) Titulaire: Roquette Frères, 62136 Lestrem (FR)
(72) Inventeur: BOIT, Baptiste, F-62400 Bethune (FR); ROSSI, Laurent, F-62000 Arras (FR)
(74) Mandataire: Cabinet Plasseraud
(86) Numéro de dépôt international: PCT/FR2013/051907
(87) Numéro de publication internationale: WO 2014/027158

(56) Documents cités:
- WO-A1-2008/147811
- WO-A2-2012/079671
- BURGER A ET AL: "Energy/temperature diagram and compression behavior of the polymorphs of D-mannitol", JOURNAL OF PHARMACEUTICAL SCIENCES, AMERICAN PHARMACEUTICAL ASSOCIATION, WASHINGTON, US, vol. 89, no. 4, 1 janvier 2000 (2000-01-01), pages 457-468, XP003002443, ISSN: 0022-3549, DOI: 10.1002/(SICI)1520-6017(200004)89:4<457::A ID-JPS3>3.0.CO;2-G cité dans la demande

## Description

La présente invention a pour objet un procédé de fabrication d'une composition particulièrement riche en D-mannitol, ce dernier étant présent sous forme de cristaux présentant un diamètre moyen volumique D4,3 supérieur à 20 µm, lesdits cristaux correspondant dans une très large mesure au polymorphe δ. Ce procédé fait appel à la technique d'évapo-cristallisation, dans laquelle on réalise la germination puis la croissance contrôlée des cristaux à des vitesses d'évaporation différentes. Au final, on dispose avantageusement de cristaux de type δ qui présentent une meilleure compressibilité que leurs homologues de type α et β et qui, de par leurs dimensions et leur pureté, conduisent à une poudre non sujette au phénomène de mottage.

Le mannitol est un polyol acyclique communément employé notamment en tant qu'excipient dans les formulations pharmaceutiques telles que les comprimés, les granules ou les poudres, ou encore en tant que stabilisant dans des compositions protéiques. Il existe trois véritables polymorphes de D-mannitol : les polymorphes α, β et δ. Par « polymorphe », on entend dans la présente Demande une structure cristalline solide de D-mannitol identifiable, comme détaillé ultérieurement, par un spectre particulier de diffraction aux rayons X. Le terme « forme » α, β ou δ pourra également être employé dans la présente Demande en lieu et place du terme « polymorphe ».

Les méthodes de détermination structurelle basées sur des données de diffraction aux rayons X sont particulièrement utiles pour étudier les polymorphes du D-mannitol et ce, pour deux raisons principales (C. E. Botez et al. Powder diffraction 2003, 18(3), 214). Tout d'abord, la détermination de la structure cristalline par rayons X permet une identification précise, directe et non équivoque d'un polymorphe donné. Ensuite, les techniques modernes d'analyse des données de diffraction permettent l'étude de profils de diffraction complexes et il est ainsi possible d'identifier le contenu exact d'une poudre composée d'un mélange de plusieurs polymorphes.

Par conséquent, cette technique est parfaitement maîtrisée par l'homme du métier et lui permet notamment de quantifier, au sein d'une composition cristalline, la proportion de chaque polymorphe. Cette quantification s'entend, par exemple, d'un pourcentage en poids d'un polymorphe donné par rapport au poids total de la composition cristalline. C'est précisément ce type de quantification qui est utilisée dans la présente Demande.

Il est connu que la cristallisation du D-mannitol conduit à la formation d'un polymorphe donné ou d'un mélange donné de polymorphes en fonction des conditions du procédé de cristallisation telles que le solvant, la concentration, la température, etc (C. E. Botez et al. Powder diffraction 2003, 18(3), 214).

Chacune des structures α, β et δ présente des caractéristiques physiques propres notamment en termes de solubilité, d'hygroscopicité, de compressibilité. A titre d'exemple, la forme β est une forme cristalline thermodynamiquement stable en conditions ambiantes standard, les formes cristallines α et δ étant des formes métastables sous ces mêmes conditions.

Une caractéristique essentielle pour un excipient est sa compressibilité : elle reflète l'aptitude dudit excipient à former des comprimés par compression directe. Les polymorphes α, β et δ de mannitol n'ont pas tous la même compressibilité (A. Bruger et al., J. Pharm. Sci. 2000, 89, 457), le polymorphe δ présentant la compressibilité la plus élevée par rapport à ses homologues α et β. Il est donc intéressant de disposer de méthodes industrielles conduisant aisément et à moindre coût à la fabrication de poudres de D-mannitol riches en polymorphe δ.

Le D-mannitol est obtenu à partir de l'hydrogénation catalytique d'un sirop de fructose qui conduit à l'obtention d'un mélange de sorbitol et de mannitol. Ensuite, un procédé de cristallisation classique permet la récupération des cristaux de D-mannitol mais on constate qu'industriellement, il conduit à la formation majoritaire du polymorphe β.

De manière alternative, de petites quantités de D-mannitol sous forme de polymorphe δ peuvent être produites par refroidissement d'une solution aqueuse de D-mannitol à 0°C puis par isolement rapide des formes δ résultantes, avant qu'elles ne se transforment en polymorphes α et β (A. Bruger et al., J. Pharm. Sci. 2000, 89, 457). Mais il s'agit encore d'un procédé dont le rendement est insuffisant pour être transposable à une échelle industrielle.

Le document WO 2012 / 079671 divulgue un procédé qui consiste à placer une solution de D-mannitol en suspension dans un gaz et à sécher le matériel afin d'obtenir des particules cristallines de D-mannitol. Le D-mannitol ainsi obtenu comprend plus de 98 % en poids de polymorphe δ, et présente un diamètre médian X50 de 200, 300 ou 450 µm (cf exemples 1, 2 et 3).

Le document WO 2008/147811 propose quant à lui un procédé qui consiste à réaliser une solution initiale de D-mannitol dans un solvant approprié tel que l'eau, à ajouter un agent auxiliaire comme le D-sorbitol, et à réaliser la solidification de D-mannitol sous forme δ. Cette solidification peut être réalisée de manière alternative par évaporation, par ajout d'un anti-solvant, par thermo-microscopie ou, préférentiellement, par refroidissement. Il est indiqué que le refroidissement de la solution ne doit pas être rapide, la vitesse de diminution de la température divulguée dans les exemples étant de 1°C par minute.

Cherchant à reproduire les essais du document précité, à des vitesses de refroidissement de 1°C par minute mais également à des vitesses plus faibles selon l'enseignement de ce document, la Demanderesse a fait le double constat suivant : non seulement les cristaux obtenus n'étaient pas systématiquement sous forme δ, mais ils présentaient un diamètre moyen volumique relativement faible, à savoir compris entre 10 µm et 20 µm.

Au regard de cette dernière caractéristique, il convient de préciser un certain nombre de points quant aux problèmes engendrés par la présence de cristaux de petites tailles. Tout d'abord, les cristaux de tailles trop faibles sont difficiles à séparer des eaux mères. Ensuite, l'opération de lavage pour éliminer les impuretés de surface est rendu plus complexe de par la petite taille des cristaux à traiter. Enfin, il est bien connu que dans le cas d'une poudre, plus la taille des cristaux est petite plus la poudre ou composition pulvérulente comprenant les dits cristaux sera sujette au phénomène de mottage. Par conséquent, le procédé tel que divulgué dans le document précité ne peut donner satisfaction.

Enfin, la Demanderesse a également connaissance d'une composition commerciale de cristaux de D-mannitol sous forme δ. Ladite composition est commercialisée par la société MERCK™ sous le nom PARTECK™ DELTA M. En analysant cette poudre, la Demanderesse a démontré qu'il ne s'agissait pas de polymorphes β uniquement : la teneur en poids sec de polymorphes δ représente environ 90 % du total des cristaux (présence concomitante de cristaux sous forme β). Partant de cette observation, la Demanderesse a alors démontré que la stabilité de ce produit à l'humidité n'était pas satisfaisante : ceci constitue un élément rédhibitoire pour un produit destiné à être mis en oeuvre comme excipient, et donc susceptible d'être conservé sur de longues périodes dans des atmosphères plus ou moins humides.

Par conséquent, il existe un besoin non encore satisfait de disposer d'un procédé de fabrication de cristaux de D-mannitol sous forme δ, présentant un rendement suffisamment important pour le rendre industrialisable, conduisant à des cristaux de haute pureté et dont le diamètre moyen volumique est au moins égal à 20 µm pour faciliter les étapes de rinçage des cristaux et éviter le phénomène de mottage de la poudre, tout en aboutissant à un produit final présentant une bonne stabilité à l'humidité. Poursuivant des recherches dans ce sens, la Demanderesse a réussi à mettre au point un tel procédé. Celui-ci repose sur la mise en oeuvre de la technique d'évapo-cristallisation, dans laquelle on contrôle à la fois l'étape de nucléation des cristaux et l'étape de croissance cristalline, en réglant les vitesses d'évaporation en fonction de la masse de la solution à évaporer. La Demanderesse est notamment parvenue à identifier les paramètres qui permettaient d'assurer le contrôle de ces 2 étapes, de manière à conduire au final à un solide ou poudre constitué à plus de 97 % en poids sec par du D-mannitol, les cristaux de D-mannitol étant représentés à plus de 98 % en poids sec par le polymorphe δ, lesdits cristaux de forme δ ayant un diamètre moyen volumique supérieur à 20 µm.

L'objet de la présente invention consiste donc en un procédé de fabrication d'une composition comprenant des cristaux de D-mannitol de forme delta dont plus de 97 %, préférentiellement plus de 98 %, très préférentiellement plus de 99 % en poids de sa matière sèche est constituée de D-mannitol, ledit procédé comprenant les étapes de :
- fabriquer une solution mère initiale de D-mannitol dans un solvant, en présence d'un agent auxiliaire,
- évaporer ledit solvant de manière à réaliser la cristallisation du D-mannitol, à travers une phase de germination et une phase de croissance des cristaux,
caractérisé en ce qu'on applique :
- pendant la phase de germination : une vitesse d'évaporation massique comprise entre 0,08 et 0,5 kg de solvant/h/kg de solution mère initiale, plus préférentiellement entre 0,1 et 0,2 kg de solvant/h/kg de solution mère initiale ;
- pendant la phase de croissance : une vitesse d'évaporation massique entre 0,005 et 0,1 kg de solvant/h/kg de solution mère initiale, plus préférentiellement entre 0,01 et 0,05 kg de solvant/h/kg de solution mère initiale.

Le terme « composition » au sens de l'invention vise aussi bien une composition pulvérulente qu'une poudre de cristaux de D-mannitol ou poudre cristalline.

La première étape dudit procédé consiste donc à fabriquer une solution mère initiale de D-mannitol dans un solvant en présence d'un agent auxiliaire, par introduction des différents constituants dans le solvant. Cette étape est préférentiellement réalisée sous agitation, et à une température permettant la dissolution complète du D-mannitol et de l'agent auxiliaire, par exemple entre 70 et 100°C.

Le solvant est tout solvant ou mélange de solvants dans lequel le D-mannitol est soluble, et dans lequel est également soluble l'agent auxiliaire. Il peut s'agir d'acétone ou d'eau, et préférentiellement d'eau.

Le D-mannitol utilisé est avantageusement celui qui est fabriqué à partir de l'hydrogénation catalytique du fructose, comme déjà discuté. Typiquement, le D-mannitol utilisé est sous forme β et présente une richesse en D-mannitol entre 10 et 99 % en poids sec, par exemple le D-mannitol PEARLITOL^{™} 50C commercialisé par la société Demanderesse.

L'agent auxiliaire est un auxiliaire de cristallisation aidant à la cristallisation du D-mannitol. L'agent auxiliaire peut être choisi parmi le sorbitol, les acides citrique et glycolique, le fructose, le mannose, les sels minéraux comme les chlorures de sodium, de potassium ou de calcium et leurs mélanges ; il s'agit préférentiellement du sorbitol, par exemple le sorbitol NEOSORB^{™} P60 commercialisé par la société Demanderesse. Le ratio D-mannitol:agent auxiliaire peut être de 20:80 à 80:20 en poids

La solution mère initiale fabriquée présente une teneur en matière sèche comprise entre 20 % et 70 % de préférence comprise entre 30 % et 60 %. Elle présente une richesse en D-mannitol comprise entre 30 % et 80 % de préférence comprise entre 40 % et 60 % en poids de matière sèche. Il est bien entendu qu'on parle ici de la solution mère initiale, telle que réalisée avant l'étape d'évaporation du solvant

La seconde étape du procédé selon l'invention consiste à évaporer le solvant, par apport de chaleur au moyen d'un échangeur de chaleur. Cette étape est réalisée sous agitation et sous vide. On applique préférentiellement un vide absolu compris entre 30 mbars et 300 mbars, de préférence compris entre 50 et 150 mbars. C'est au cours de cette étape qu'a lieu la cristallisation du D-mannitol en 2 temps ou 2 phases : il y a tout d'abord apparition des cristaux (c'est la phase de germination) puis développement de ces cristaux (c'est la phase de croissance cristalline).

C'est de manière visuelle qu'on fait la distinction entre ces 2 phases : l'apparition de cristaux visibles à l'oeil nu (blanchiment de la solution) correspond à la transition entre l'étape de germination et celle de croissance. Cet instant marque le changement des conditions opératoires telles que définies plus haut, c'est-à-dire la diminution de la vitesse d'évaporation massique. Préférentiellement, on choisit d'attendre un temps compris entre 5 et 15 minutes à partir de l'apparition des premiers cristaux avant de modifier la vitesse d'évaporation. Pour l'homme du métier habitué à conduire des expériences de cristallisation, la distinction entre ces 2 phases relève de la routine. Qui plus est, des expériences réalisées par la Demanderesse à 2 échelles différentes ont démontré la très grande facilité avec laquelle on pouvait identifier ces 2 phases, tant au niveau d'un test de laboratoire que d'une expérimentation dite « pilote ».

De manière plus précise, pendant la phase de germination, on applique une vitesse d'évaporation massique comprise entre 0,08 et 0,5 kg de solvant/h/kg de solution mère initiale, plus préférentiellement entre 0,1 et 0,2 kg de solvant/h/kg de solution mère initiale.

De manière pratique, l'homme du métier impose une vitesse d'évaporation en jouant sur la température de l'échangeur de chaleur : typiquement, en réglant la température d'un serpentin relié à un bain d'huile thermostaté. A partir de simples tests de routine, il parvient à déterminer la température ad hoc conduisant à une vitesse d'évaporation massique donnée.

De manière plus précise, pendant la phase de croissance, on applique une vitesse d'évaporation massique entre 0,005 et 0,1 kg de solvant/h/kg de solution mère initiale, plus préférentiellement entre 0,01 et 0,05 kg de solvant/h/kg de solution mère initiale. Une des clés de la présente invention repose donc sur le choix de cette double rampe de vitesse d'évaporation : rapide pendant la phase de germination, plus lente pendant la phase de croissance cristalline.

Plus particulièrement, l'invention a pour objet un procédé de fabrication d'une composition, comprenant des cristaux de D-mannitol de forme delta (δ), dont plus de 97 %, préférentiellement plus de 98 %, très préférentiellement plus de 99 % en poids de sa matière sèche est constituée de D-mannitol, ledit procédé comprenant les étapes suivantes :
- fabriquer une solution mère initiale de D-mannitol dans un solvant, en présence d'un agent auxiliaire,
- évaporer ledit solvant de manière à réaliser la cristallisation du D-mannitol, à travers une phase de germination et une phase de croissance des cristaux,
caractérisé en ce qu'on applique
- pendant la phase de germination : une vitesse d'évaporation massique comprise entre 0,08 et 0,5 kg de solvant/h/kg de solution mère initiale, plus préférentiellement entre 0,1 et 0,2 kg de solvant/h/kg de solution mère initiale ;
- pendant la phase de croissance : une vitesse d'évaporation massique entre 0,005 et 0,1 kg de solvant/h/kg de solution mère initiale, plus préférentiellement entre 0,01 et 0,05 kg de solvant/h/kg de solution mère initiale.
et en ce que
- l'étape d'évaporation est réalisée sous agitation et sous un vide absolu compris entre 50 mbars et 100 mbars;
- le solvant est de l'eau ;
- l'agent auxiliaire est le sorbitol.

En outre, le procédé selon l'invention comprend une étape de séparation des cristaux de la solution, notamment par centrifugation ou essorage. Cette étape est ensuite suivie d'une étape de clairçage (rinçage) qui consiste à nettoyer en surface les cristaux fabriqués, en les rinçant dans un solvant. Préférentiellement, cette étape consiste à effectuer un premier rinçage à l'eau froide (eau dont la température est comprise entre 5 °C et 25 °C), puis un second rinçage dans un mélange d'eau et d'éthanol.

Enfin, le procédé selon l'invention comprend une étape de séchage, visant à évaporer le solvant de la composition fabriquée.

Le procédé objet de l'invention présente notamment pour avantage de permettre la production avec un rendement élevé et un haut degré de pureté d'une composition de D-mannitol majoritairement sous forme de cristaux de forme delta (δ), en particulier d'une composition pulvérulente ou poudre de D-mannitol majoritairement sous forme de cristaux de forme delta (δ) (plus de 98 % en poids sec des cristaux de D- mannitol), les dits cristaux présentant un diamètre moyen volumique D4,3 supérieur à 20 µm, préférentiellement un diamètre moyen volumique compris entre 30 µm et 100 µm, très préférentiellement entre 50 µm et 100 µm.

Une composition est divulguée comprenant des cristaux de D-mannitol, dont plus de 97 %, préférentiellement plus de 98 %, très préférentiellement plus de 99 % en poids de sa matière sèche est constituée de D-mannitol, et caractérisée en ce que les cristaux de D-mannitol contiennent plus de 98 %, préférentiellement plus de 99 %, très préférentiellement plus de 99,5 % en poids sec de polymorphes δ. Comme déjà indiqué, ces % en polymorphes δ sont déterminés par diffraction aux rayons X, technique parfaitement connue et maîtrisée permettant la quantification des différentes populations cristallines au sein d'une poudre.

Cette composition est aussi caractérisée en ce que lesdits cristaux ont un diamètre moyen volumique D4,3 supérieur à 20 µm, préférentiellement un diamètre moyen volumique compris entre 30 µm et 100 µm, très préférentiellement entre 50 µm et 100 µm. Ce diamètre moyen volumique (moyenne arithmétique) D4,3 est déterminé sur un granulomètre à diffraction Laser type LS 230 de la société BECKMAN-COULTER™, équipé de son module de mesure en voie liquide, en suivant le manuel technique et les spécifications du constructeur. Les cristaux sont dispersés dans l'éthanol absolu avec du tensio-actif (notamment de type « twin » commercialisé par TEGO™) _{.}

Cette composition est notamment celle susceptible d'être obtenue à partir du procédé décrit ci-dessus.

La composition, en particulier la composition pulvérulente ou la poudre, comprend des cristaux de D-mannitol susceptible d'être obtenue selon le procédé décrit ci-dessus.

La composition est constituée de plus de 97 %, préférentiellement plus de 98 %, très préférentiellement plus de 99 % de sa matière sèche de D-mannitol et est notamment caractérisée en ce que les cristaux contiennent plus de 98 %, préférentiellement plus de 99 %, très préférentiellement plus de 99,5 % en poids sec de polymorphe δ, et en ce que lesdits cristaux ont un diamètre moyen volumique D4,3 supérieur à 20 µm, préférentiellement un diamètre moyen volumique compris entre 30 µm et 100 µm, très préférentiellement entre 50 µm et 100 µm.

Cette composition présente notamment pour avantage d'être très compressible, non sujette au phénomène de mottage et stable dans le temps à l'humidité.

Les exemples qui suivent permettent de mieux appréhender la présente invention, sans pour autant en limiter la portée.

### EXEMPLES

### Exemple 1

Cet exemple illustre l'art antérieur, et plus précisément les cristaux tels qu'obtenus dans les conditions décrites et revendiquées dans le document WO 2008 / 147811. On commence par réaliser une solution mère aqueuse de D-mannitol (PEARLITOL™ 50C commercialisé par la société Demanderesse) et de D-sorbitol (NEOSORB™ P60 également commercialisé par la Demanderesse). Ce mélange est réalisé par introduction, dans un bêcher thermostaté à double enveloppe de 150 mL, de 20 mL d'eau, puis du D-mannitol puis du D-sorbitol. Le mélange est réalisé sous agitation (barreau magnétique à une vitesse de rotation de 50 tours / min), à une température de 80 °C.

Dans les essais n° 1, 4 et 6, on a appliqué une vitesse de refroidissement de 1°C par minute, comme décrit dans le document WO 2008/147811. Comme ce document mentionne aussi que cette vitesse ne doit pas être rapide, on a également réalisé des tests à 0,1 °C / min (essais n° 2, 5 et 7) et même à 0,05 °C /min (essai n° 8) et 0,01 °C / min (essai n° 5). Les cristaux obtenus sont récupérés sur un filtre Milliport de 0,45 µm. La forme cristalline est analysée par diffraction aux rayons X. On détermine également la valeur du diamètre moyen volumique. Ces paramètres de même que les caractéristiques de la solution initiale et les vitesses de refroidissement apparaissent dans le tableau 1.

Dans chacun des 8 essais, on a obtenu un polymorphe majoritaire (plus de 99 % en poids des cristaux étant constitué par ce polymorphe). Il apparaît tout d'abord que, dans certaines conditions (essais n° 3, 5 et 8), on n'aboutit pas à la forme δ mais à la forme β : le procédé selon le document WO 2008/147811 ne conduit donc pas de manière certaine à la forme souhaitée. De plus, lorsqu'on est bien en présence de la forme δ majoritaire (essais n° 1, 2, 4, 6, 7), le diamètre moyen volumique des cristaux est systématiquement inférieur à 20 µm.

**Tableau 1**

| Essai n° | 1 | 2 | 3 | 4 | 5 | 6 | 7 | 8 |
|---|---|---|---|---|---|---|---|---|
| Ratio (poids | 42 | 42 | 42 | 62 | 62 | 50 | 50 | 50 |
| sec/sec) | / | / | / | / | / | / | / | / |
| mannitol/ sorbitol | 58 | 58 | 58 | 38 | 38 | 50 | 50 | 50 |
| MS % | 39,5 | 39,5 | 39,5 | 39,7 | 39,7 | 50 | 50 | 50 |
| Vitesse °C/min | 1 | 0,1 | 0,01 | 1 | 0,1 | 1 | 0,1 | 0,05 |
| Forme cristalline (RX)* | delta | delta | beta | delta | beta | delta | delta | beta |
| Diamètre moyen volumique (µm) | 10 | 13 | Non mesuré | 14 | Non mesuré | 16 | 19 | Non mesuré |

| | | | | | | | | |
|---|---|---|---|---|---|---|---|---|
| * forme présente à plus de 99 % (en poids) | | | | | | | | |

### Exemple 2

Cet exemple a pour but d'illustrer, à l'échelle du laboratoire, l'influence des vitesses d'évaporation massiques au cours des étapes de germination et de croissance, dans un procédé de fabrication de D-mannitol, dans des conditions selon l'invention et hors invention.

On prépare tout d'abord une solution mère à 50% de matière sèche contenant 50 % en poids sur sec de D-mannitol PEARLITOL™ 50C et 50 % en poids sur sec de sorbitol NEOSORB™ P60. La solution mère initiale est chauffée à 80°C de manière à éliminer toutes traces de cristaux.

### Essai n° 9

Cet essai illustre l'invention. 5 kg de la solution mère initiale sont chargés dans un évapocristallisoir de laboratoire de volume égal à 5 L équipé d'un serpentin relié à un bain d'huile thermostaté. L'agitation est réalisée par une turbine Rushton (150 tours / minute). On régule le vide à 70 mbars. La température de l'échangeur de l'évapocristallisoir est réglée de manière à obtenir une vitesse d'évaporation massique égale à 0,14 kg d'eau/h/kg de solution mère initiale. Cette vitesse d'évaporation est maintenue jusqu'à 10 minutes après l'apparition des premiers cristaux. La vitesse d'évaporation est ensuite réduite à environ 0,012 kg d'eau/h/kg de solution mère initiale. La manipulation est arrêtée lorsque 1 kg d'eau est évaporé. Les cristaux, la poudre ou composition comprenant les cristaux sont ensuite récupérés, lavés à l'eau froide puis avec un mélange éthanol/eau (95 % / 5 % en poids de chaque constituant) et séchés en étuve.

### Essais n° 10 et 11

Ils illustrent des domaines hors invention, et sont réalisés dans les mêmes conditions que l'essai précédent. Seules varient les vitesses d'évaporation.

**Tableau 2**

| Essai n° | 9 | 10 | 11 |
|---|---|---|---|
| Invention / Hors Invention | IN | HI | HI |
| vitesse d'évaporation phase de germination (kg d'eau/h/kg de solution mère initiale) | 0,14 | 0,14 | 0,012 |
| vitesse d'évaporation phase de croissance (kg d'eau/h/kg de solution mère initiale) | 0,012 | 0,14 | 0,012 |
| Masse évaporée fin (kg) | 1 | 1 | 1 |
| Forme cristalline RX* | delta | delta | beta |
| Teneur en mannitol (% en poids sec) | 98,7 | 97,3 | Non mesuré |
| Diamètre moyen volumique (µm) | 40 | 15 | Non mesuré |

| | | | |
|---|---|---|---|
| * forme présente à plus de 99 % (en poids) | | | |

On démontre bien que, par application des vitesses d'évaporation selon l'invention pendant la phase de germination et pendant celle de croissance, on aboutit à des polymorphes δ et à des cristaux présentant un diamètre moyen volumique supérieur à 20 µm.

### Exemple 3

Cet exemple a pour but d'illustrer, à l'échelle d'un pilote industriel, l'influence des vitesses d'évaporation massiques au cours des étapes de germination et de croissance, dans un procédé de fabrication de D-mannitol, dans des conditions selon l'invention et hors invention.

### Essais n° 12 à 14

Ces essais illustrent l'invention.

On réalise les mêmes opérations que précédemment, mais sur un appareil pilote de 500 L de volume utile, et avec 300 kg de solution mère. Le vide est toujours de 70 mbars. L'agitation est réalisée par une turbine Rushton (150 tours / minute). Les condensats sont récupérés dans une cuve sur peson. La séparation et les étapes de lavage à l'eau froide puis avec un mélange éthanol/eau (95 % / 5 % en poids de chaque constituant) sont réalisées sur une turbine Rousselet Robatel EHR 501 G et le séchage avec un lit d'air fluidisé.

### Essai n° 15 et 16

Ils illustrent des domaines hors invention, et sont réalisés dans les mêmes conditions que les essais 12 à 14. Seules varient les vitesses d'évaporation.

Les résultats du tableau 3 démontrent que c'est bien la double sélection réalisée sur les vitesses d'évaporation, tant au niveau de l'étape de germination que de celle de croissance, qui conduit à une composition comprenant des cristaux de haute pureté en D-mannitol, extrêmement riches en polymorphe δ, et présentant un diamètre moyen volumique supérieur à 20 µm.

**Tableau 3**

| Essai n° | 12 | 13 | 14 | 15 | 16 |
|---|---|---|---|---|---|
| Invention / Hors Invention | IN | IN | IN | HI | HI |
| vitesse d'évaporation phase de germination (kg d'eau/h/kg de solution mère initiale) | 0,133 | 0,133 | 0,133 | 0,133 | 0,133 |
| vitesse d'évaporation phase de croissance (kg d'eau/h/kg de solution mère initiale) | 0,033 | 0,05 | 0,015 | 0,001 | 0,15 |
| Masse évaporée fin (kg) | 85 | 85 | 85 | 85 | 85 |
| Forme cristalline RX* | delta | delta | delta | beta | delta |
| Teneur en mannitol (% en poids sec) | 99,5 | 99,0 | 99,5 | Non mesuré | 97,5 |
| Diamètre moyen volumique (µm) | 80 | 60 | 97 | Non mesuré | 15 |

| | | | | | |
|---|---|---|---|---|---|
| * forme présente à plus de 99 % (en poids) | | | | | |

### Exemple 4

Dans cet exemple, on compare la stabilité à l'humidité d'un produit selon l'invention et du produit commercialisé par la société MERCK™ sous la dénomination PARTECK™ DELTA M.

En premier lieu, une analyse aux RX des cristaux contenus dans ce dernier produit démontre la présence d'environ 90 % de polymorphes δ.

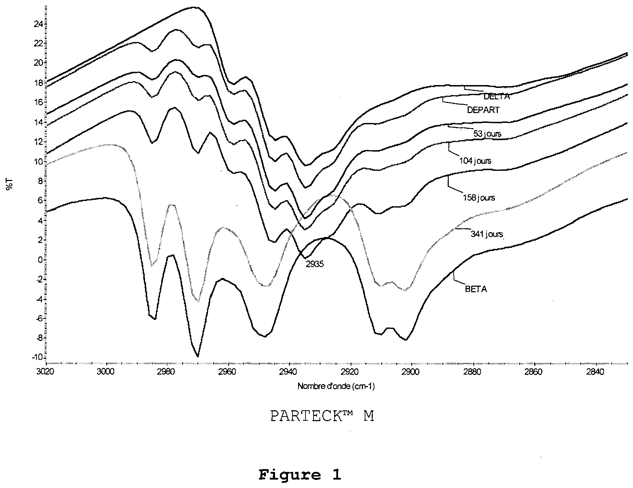

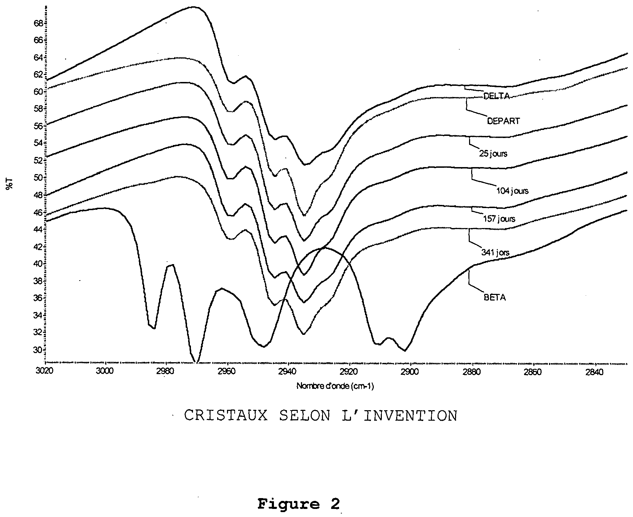

Ensuite, on stocke ce produit et la poudre ou composition issue de l'essai selon l'invention n° 12 dans une enceinte climatisée (20 °C) et à un degré d'humidité relative contrôlé (75 %). On réalise sur ces 2 produits des spectres qualitatifs IR à différents instants, de manière à suivre l'évolution des formes cristallines δ vers les formes β. Ces spectres sont représentés sur les figures 1 / 2 et 2 / 2µ respectivement pour le produit commercial et pour le produit selon l'invention.

Il apparaît que le produit commercial est complètement passé sous forme β au bout de 341 jours, ce qui n'est pas le cas du produit selon l'invention : ce dernier présente donc une plus grande stabilité à l'humidité. Ce résultat est particulièrement intéressant en vue d'une application dans le domaine pharmaceutique : en disposant d'une forme δ stable dans le temps, on pourra fabriquer des granulés à la compressibilité améliorée et prolongée dans le temps, sans affecter la bio-disponibilité du principe actif contenu dans ledit granulé.

## Revendications

1. Procédé de fabrication d'une composition comprenant des cristaux de D-mannitol dont plus de 97 %, préférentiellement plus de 98 %, très préférentiellement plus de 99 % de sa matière sèche est constituée de D-mannitol, ledit procédé comprenant les étapes de :
- fabriquer une solution mère initiale de D-mannitol dans un solvant, et en présence d'un agent auxiliaire,
- évaporer ledit solvant de manière à réaliser la cristallisation du D-mannitol, à travers une phase de germination et une phase de croissance des cristaux,
**caractérisé en ce qu'**on applique :
- pendant la phase de germination : une vitesse d'évaporation massique comprise entre 0,08 et 0,5 kg de solvant/h/kg de solution mère initiale, plus préférentiellement entre 0,1 et 0,2 kg de solvant/h/kg de solution mère initiale ;
- pendant la phase de croissance : une vitesse d'évaporation massique entre 0,005 et 0,1 kg de solvant/h/kg de solution mère initiale, plus préférentiellement entre 0,01 et 0,05 kg de solvant/h/kg de solution mère initiale
et **en ce que**
- l'étape d'évaporation est réalisée sous agitation et sous un vide absolu compris entre 50 mbars et 100 mbars;
- le solvant est de l'eau ;
- l'agent auxiliaire est le sorbitol.

2. Procédé selon la revendication 1, **caractérisé en ce que** la fabrication de la solution de D-mannitol dans le solvant et en présence de l'agent auxiliaire est réalisée par mélange des constituants sous agitation à une température permettant la dissolution complète du D-mannitol et de l'agent auxiliaire.

3. Procédé selon l'une des revendications 1 ou 2, **caractérisé en ce que** la fabrication de la solution mère initiale de D-mannitol est réalisée à une température comprise entre 70 °C et 100 °C.

4. Procédé selon l'une des revendications 1 à 3, **caractérisé en ce que** la solution mère initiale présente une teneur en matière sèche comprise entre 20 % et 70 %, de préférence comprise entre 30 % et 60 % en poids.

5. Procédé selon l'une des revendications 1 à 4, **caractérisé en ce que** la solution mère initiale présente une richesse en D-mannitol comprise entre 30 % et 80 %, de préférence comprise entre 40 % et 60 % en poids de matière sèche.

6. Procédé selon l'une des revendications 1 à 5, **caractérisé en ce qu'**il comprend une étape de clairçage consistant à effectuer un premier rinçage à l'eau froide, puis un second rinçage dans une solution d'eau et d'éthanol.

7. Procédé selon l'une des revendications 1 à 6, **caractérisé en ce qu'**il comprend une étape de séchage.

## Patentansprüche

1. Verfahren zur Herstellung einer Zusammensetzung, umfassend D-Mannitol Kristalle, wobei mehr als 97 %, bevorzugt mehr als 98 %, sehr bevorzugt mehr als 99 % der Trockenmasse aus D-Mannitol bestehen, wobei das Verfahren die Schritte umfasst:
- Herstellen einer D-Mannitol Ausgangsstammlösung in einem Lösungsmittel und in Gegenwart eines Hilfsmittels,
- Verdampfen des Lösungsmittels, um die Kristallisation des D-Mannitols durch eine Keimphase und eine Kristallwachstumsphase zu realisieren,
**dadurch gekennzeichnet, dass**:
- während der Keimphase: eine Massenverdampfungsgeschwindigkeit zwischen 0,08 und 0,5 kg Lösungsmittel/h/kg der Ausgangsstammlösung, mehr bevorzugt zwischen 0,1 und 0,2 kg Lösungsmittel/h/kg der Ausgangsstammlösung,
- während der Wachstumsphase: eine Massenverdampfungsgeschwindigkeit zwischen 0,005 und 0,1 kg Lösungsmittel/h/kg der Ausgangsstammlösung, mehr bevorzugt zwischen 0,01 und 0,05 kg Lösungsmittel/h/kg der Ausgangsstammlösung
angewendet wird und, dass
der Verdampfungsschritt unter Bewegung und unter vollem Vakuum zwischen 50 mbar und 100 mbar realisiert wird;
- das Lösungsmittel Wasser ist,
- das Hilfsmittel Sorbitol ist.

2. Verfahren nach Anspruch 1, **dadurch gekennzeichnet, dass** die Herstellung der D-Mannitol Lösung in dem Lösungsmittel und in Gegenwart des Hilfsmittels durch Vermischen der Bestandteile unter Bewegung bei einer Temperatur, welche die vollständige Auflösung von D-Mannitol und dem Hilfsmittel ermöglicht, realisiert wird.

3. Verfahren nach Anspruch 1 oder 2, **dadurch gekennzeichnet, dass** die Herstellung der D-Mannitol Ausgangsstammlösung bei einer Temperatur zwischen 70ºC und 100ºC realisiert wird.

4. Verfahren nach einem der Ansprüche 1 bis 3, **dadurch gekennzeichnet, dass** die Ausgangsstammlösung einen Trockenmassengehalt zwischen 20 Gew.-% und 70 Gew.-%, bevorzugt zwischen 30 Gew.-% und 60 Gew.-% aufweist.

5. Verfahren nach einem der Ansprüche 1 bis 4, **dadurch gekennzeichnet, dass** die Ausgangsstammlösung einen reichen D-Mannitol-Gehalt zwischen 30 Gew.-% und 80 Gew.-%, bevorzugt zwischen 40 Gew.-% und 60 Gew.-% der Trockenmasse aufweist.

6. Verfahren nach einem der Ansprüche 1 bis 5, **dadurch gekennzeichnet, dass** es einen Spülschritt umfasst, der aus einer ersten Spülung mit kaltem Wasser danach einer zweiten Spülung in einer Lösung von Wasser und Ethanol besteht.

7. Verfahren nach einem der Ansprüche 1 bis 6, **dadurch gekennzeichnet, dass** es einen Trocknungsschritt umfasst.

## Claims

1. A process for producing a composition comprising D-mannitol crystals, of which more than 97%, preferentially more than 98% and very preferentially more than 99% of the dry matter thereof consists of D-mannitol, said process comprising the steps of:
- producing an initial stock solution of D-mannitol in a solvent, and in the presence of an auxiliary agent,
- evaporating said solvent so as to carry out the crystallization of the D-mannitol, through a seeding phase and a crystal growth phase,
**characterized in that**:
- during the seeding phase: an evaporation rate by mass of between 0.08 and 0.5 kg of solvent/h/kg of initial stock solution, more preferentially between 0.1 and 0.2 kg of solvent/h/kg of initial stock solution, is applied;
- during the growth phase: an evaporation rate by mass of between 0.005 and 0.1 kg of solvent/h/kg of initial stock solution, more preferentially between 0.01 and 0.05 kg of solvent/h/kg of initial stock solution, is applied,
and **in that**
- the evaporation step is carried out with stirring and under an absolute vacuum of between 50 mbar and 100 mbar;
- the solvent is water;
- the auxiliary agent is sorbitol.

2. The process as claimed in claim 1, **characterized in that** the production of the solution of D-mannitol in the solvent and in the presence of the auxiliary agent is carried out by mixing the constituents with stirring at a temperature which allows complete dissolution of the D-mannitol and of the auxiliary agent.

3. The process as claimed in either of claims 1 and 2, **characterized in that** the production of the initial stock solution of D-mannitol is carried out at a temperature of between 70°C and 100°C.

4. The process as claimed in one of claims 1 to 3, **characterized in that** the initial stock solution has a dry matter content of between 20% and 70% and preferably of between 30% and 60% by weight.

5. The process as claimed in one of claims 1 to 4, **characterized in that** the initial stock solution has a D-mannitol richness of between 30% and 80% and preferably of between 40% and 60% by weight of dry matter.

6. The process as claimed in one of claims 1 to 5, **characterized in that** it comprises a clarifying step consisting in carrying out a first rinsing with cold water, and then a second rinsing in a solution of water and ethanol.

7. The process as claimed in one of claims 1 to 6, **characterized in that** it comprises a drying step.
